# EUROPEAN PATENT SPECIFICATION

(11) **EP 0 361 282 B1**
(45) Date of publication and mention of the grant of the patent: **18.08.1993**
(21) Application number: 89117306.4
(22) Date of filing: 19.09.1989
(51) Int. Cl.: C07C 19/08, C07C 17/00

(54) **Branched perfluoroalkyl halides and process for the preparation thereof**
Verzweigte Perfluoralkylhalogenide und Verfahren zu ihrer Herstellung
Halogénures de perfluoroalkyle ramifié et procédé pour leur préparation

(30) Priority: 20.09.1988 IT 2200588
(43) Date of publication of application: 04.04.1990
(73) Proprietor: AUSIMONT S.p.A., I-20121 Milano (IT)
(72) Inventor: Tonelli, Claudio, Dr., I-20049 Concorezzo (Milano) (IT); Tortelli, Vito, Dr., I-20137 Milano (IT)
(74) Representative: Barz, Peter, Dr.

(56) References cited:
- DE-A- 3 600 108
- US-A- 4 097 344
- US-A- 4 535 184
- CHEMICAL ABSTRACTS, vol. 96, no. 9, March 1, 1982, Columbus, Ohio, USA COE et al. "Reactions of tetrafluorethykebe oligomers. Part I. Some pyrolytic reactions of the pentamer and hexamer and of the fluorine adducts of the tetramer and pentamer" pages 548-549, abstract-no. 68 245w
- CHEMICAL ABSTRACTS, vol.102, no. 17, April 29, 1985, Columbus, Ohio, USA COE et al. "Polyfluoro-1,2 -epoxyalkanes and cyclo- alkanes. Part IV. Thermal reactions of the epoxides of the pentamer and hexamer oligomers of tetrafluoro- ethene" page 584, abstract-no. 149 005n
- CHEMICAL ABSTRACTS, vol. 101, no. 7, August 13, 1984, Columbus, Ohio, USA ADCOCK et al. "Aerosol direct fluorination: alkyl halides. 2. Chlorine shifts and the stability of radicals" page 548, abstract-no. 54 188q
- CHEMICAL ABSTRACTS, vol. 85, no. 15, October 11, 1976, Columbus, Ohio, USA HASZELDINE et al. "Addition of free radicals to unsaturated systems. Part XXII. Photochemical addition of trifluoroiodomethane and iodine to perfluoro-(3 -methylbut-1-ene) and photochemical dimerization of the olefin" page 426, abstract-no. 108 242z

## Description

Object of the present invention are perfluoroalkyl halides wherein the carbon atom linked to the halogen atom is of the secondary or tertiary type, said halides having one of the general formulae (1) to (5): 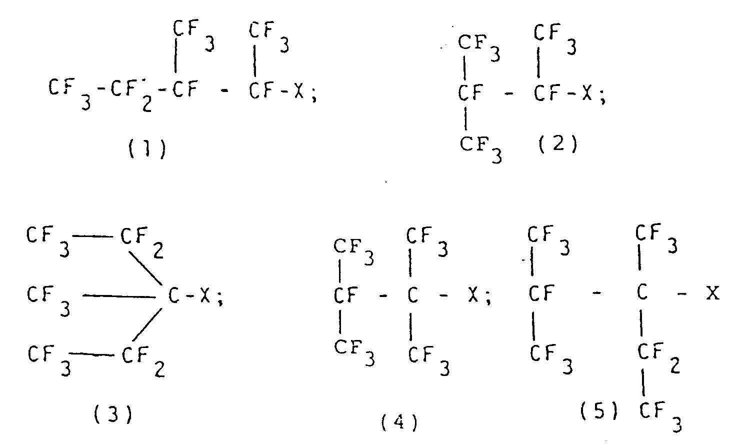
wherein X is I, Cl, Br; provided that in formula (2) X is only Br and that in formulae (3) and (4) X is not Br.

Also provided is a process for preparing, among others, the above compounds, i.e., a process for preparing branched perfluoroalkyl halides wherein the carbon atom linked to the halogen atom is of the tertiary or secondary type, comprising the thermal decomposition, in the presence of a halogen of formula X₂ (X = Br, Cl or I) and at a temperature ranging from 90° to 250°C, of a branched perfluoroalkane containing at least one tertiary carbon atom and one quaternary carbon atom or two adjacent quaternary carbon atoms.

The above perfluoroalkyl halides are products of great interest in that they are utilizable in a plurality of fields.

The perfluoroalkyl chlorides can advantageously be employed as solvents and cooling liquids whereas the corresponding bromides and iodides may find use as telogens in radical-induced telomerization reactions.

The tertiary perfluoroalkyl iodides, owing to the lability of the C-I bond, can be utilized in lasers obtained by means of photoiododissociation, where the iodine atom is excited by the action of sunlight (EP-A-288,701).

Branched perfluoroalkyl halides are products which cannot be synthesized easily. In fact, the known processes for preparing said compounds require toxic and expensive reagents such as IF₅ or IF and in most cases the yields of desired products are low.

For example, J.A. Young and Th.M. Reed in J. Org. Chem., 32 (1967), page 1682, describe the preparation of 2-iodoperfluoro-2-methylpropane (which is the branched perfluoroalkyl halide having the lowest number of carbon atoms) by reaction of IF with perfluoroisobutylene during 60 hours at 130°C.

H. Fainberg and Braid in Journal of American Chemical Society describe the use, instead of IF, of a mixture of IF₅ and iodine in order to obtain the above compound. Under the just described conditions it is, however, not possible to obtain long-chain, branched perfluoroalkyl iodides, even if large amounts of catalysts, such as CoF₃ and SbF₅, are added.

Another method for obtaining 2-iodoperfluoromethylpropane is described by Mochalina, Galachor, Knuyant in CA 66 (1967) 45.901y and is based on the reaction, in a steel autoclave, of perfluoroisobutylene with iodine and KF in the presence of a solvent such as nitrobenzene, said reaction being conducted for 10 hours at 180°C. Said method allows to obtain the above compound in high yields. However, also this process is not capable of providing long-chain branched perfluoroalkyl iodides or cannot be conducted in the absence of solvent.

According to EP-A-288,701, branched perfluoroalkanes of formula CF₃(CF₂)ₙC(CF₃)₂I, wherein n = 1 or 2, can be prepared by reacting the corresponding perfluoroolefins with a mixture of I₂ and IF₅ (molar ratio 2:1) and at least 1 mole of a metal fluoride selected from LiF, AgF, CuF, KF, RbF and CsF, at temperatures ranging from 170°C to 220°C. In said process the perfluoroolefin must be present in a molar amount which is five times higher than the molar amount of IF₅.

In order to obtain a high yield of the desired product it is necessary to employ an excess of metal fluoride with respect to the stoichiometric amount necessary for the reaction.

The excess metal fluoride must be removed from the crude reaction product which, of course, requires further steps such as, e.g., washing of the crude reaction product with alkaline water, separation of the organic phase and subsequent drying before carrying out a fractionated distillation of the crude material.

Thus, there is a need to have available a process capable of providing branched perfluoroalkyl halides which does not show the drawback of the known processes.

It is known that in perfluoroalkane structures the strength of the C-C bond decreases as the substitution of the fluorine atoms by perfluoroalkyl groups increases. For example, it is disclosed in J. Fluorine Chem. 1981, p. 417ff (CA 96(1982), p. 548/49; 68245w) that the pyrolysis of certain fluorinated oligomers of tetrafluoroethylene over glass beads at temperatures around 500°C caused preferential thermolysis of C-C bonds at points of maximum substitution.

Published PCT patent application WO88/08007 in the name of the applicant describes branched perfluoroalkanes having at least one quaternary carbon atom and one tertiary carbon atom or two adjacent quaternary carbon atoms.

Said compounds, although being stable at room temperature, give rise, by thermal reaction, to decomposition products resulting from the homolytic scission of the C-C bond between the tertiary carbon atom and the quaternary carbon atom or between the two quaternary carbon atoms.

It has now, surprisingly, been found that it is possible to obtain, in quantitative yields, branched perfluoroalkyl halides characterized by a tertiary and/or secondary carbon atom bound to the halide, by thermal decomposition, at temperatures ranging from 90°C to 250°C, preferably from 130°C to 180°C, in the presence of a halogen of formula X₂, (X = Br, Cl or I) of branched perfluoroalkanes containing at least one tertiary carbon atom and one quaternary carbon atom or two adjacent quaternary carbon atoms.

This result is quite unexpected as it was not forseeable that the perfluoroalkyl halide formation rate is so high and that the reaction is so selective towards the perfluoroalkyl halides.

A confirmation of what has been found is obtained by comparing, for example, the kinetic rate constant of the decomposition of the starting perfluoroalkane 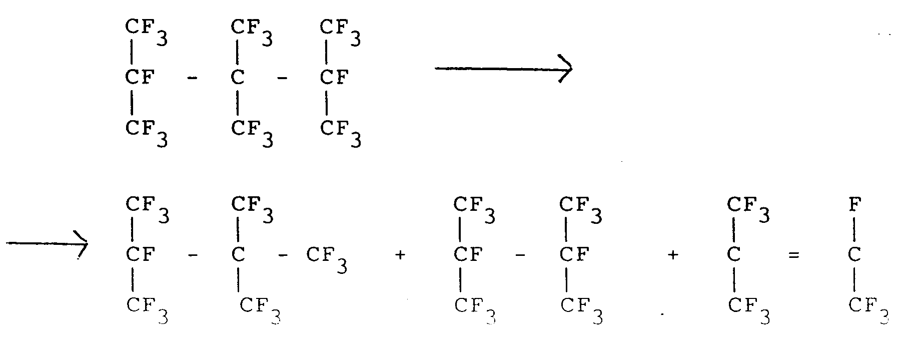
(1.2.10⁻⁵ at 150°C; 4.3.10⁻⁵ at 160°C) with the kinetic rate constant of the formation reaction, starting from the same substrate, of the perfluoroalkyl halides of formula: 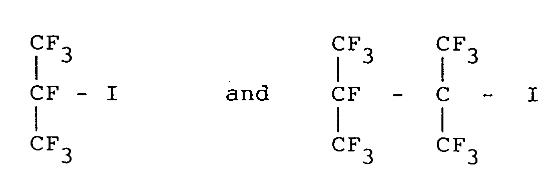
(2.3.10⁻⁵ at 150°C and 7.4.10⁻⁵ at 160°C).

As can be deduced from the above values, the rate of formation of the above products is about two times the rate of the decomposition of the starting material in the absence of halogens.

The process according to the present invention not only allows to obtain the new branched perfluoroalkyl halides according to the invention, but also represents an alternative process for the production of known compounds which so far have been prepared by the above processes of the prior art, such as for example: 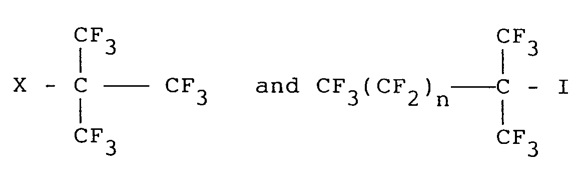
wherein n is 1 or 2.

The perfluoroalkanes utilized as starting materials can be selected, for example, from:
(A) 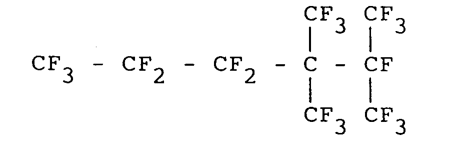 (Chemical Abstracts Vol. 101, 121530). The perfluoroalkyl halides obtainable from perfluoroalkane (A) according to the process of the present invention are the following: 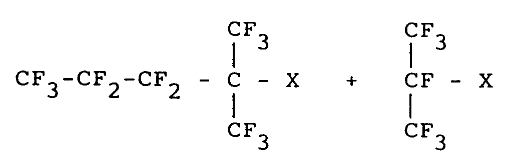 When X = I, the product having the higher number of carbon atoms is the product obtained according to EP-A-228,701, while the other products (X = I or Br) are known from EP-A-142,041.
(B) 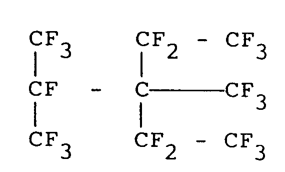 obtainable according to US-A-3,962,358.
   The products obtainable from perfluoroalkane (B) according to the process of the present invention are: 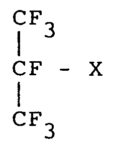 and the product of formula (3) above.
(C) 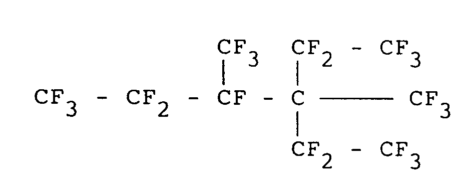 obtainable according to US-A-3,950,235.
   The products obtainable from perfluoroalkane (C) according to the process of the invention are the following: 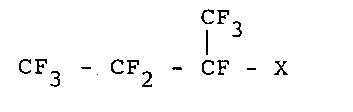 and the above product (3).
(D) 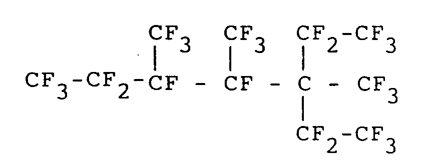 obtainable according to US-A-3,950,235.
   The perfluoroalkyl halides obtainable from perfluoroalkane (D) according to the process of the invention are the product of formula (1) above and the already mentioned product (3).
(E) 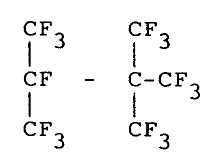 The products obtainable from perfluoroalkane (E) according to the process of the invention are the following: 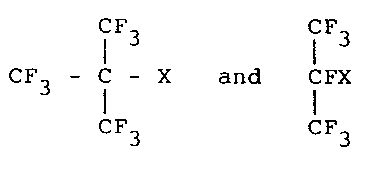
(F) 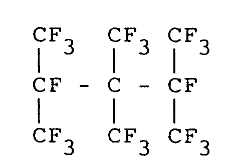 preparable according to WO 88/08007. The products obtainable according to the process of the invention when starting from perfluoroalkane (F) are compound (4) and the already mentioned product of formula:
(G) 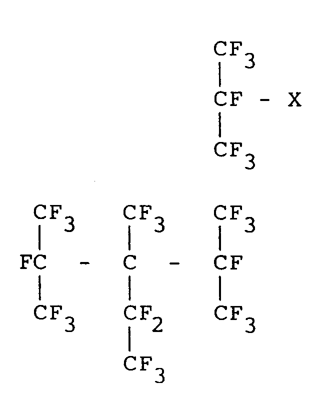 obtainable according to WO 88/8007. According to the process of the invention, from perfluoroalkane (G), product (5) and the already cited product 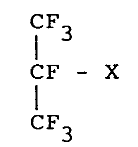 may be obtained.
(H) 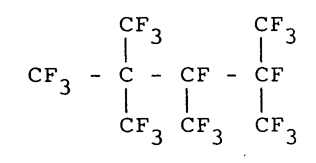 The perfluoroalkyl halides obtainable from perfluoroalkane (H) are XC(CF₃)₃ and the above product (2).
   The amount of X₂ preferably is at least stoichiometric.
   Upon completion of the reaction, the reaction products may be isolated from the crude reaction product merely by fractionated distillation. They are easy to separate since their boiling points differ substantially.

The following examples are given in order to illustrate the present invention without, however, limiting it.

### EXAMPLE 1 (comparative)

Into a 50 ml glass autoclave 20 g of perfluoroalkane (F) were introduced. The temperature was raised to 161°C and maintained for 10 hours, whereafter the product mixture was cooled and discharged. NMR and IR analyses revealed that said mixture contained, apart from the starting material, the following compounds: 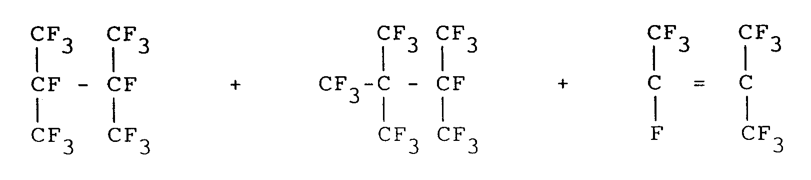
Said procedure was repeated at different temperatures, withdrawing samples at certain time intervals which allowed to calculate k_{dec} as a function of the temperature:

| T°C | k_{dec} |
|---|---|
| 141 | 2.9.10⁻⁶ |
| 151 | 1.2.10⁻⁵ |
| 161 | 4.3.10⁻⁵ |

### EXAMPLE 2

The autoclave used in the preceding example was charged with 20 g of the branched perfluoroalkane of example 1 (0.041 moles) whereafter 31 g of I₂ (0.122 moles) were introduced and the mixture was heated to 161°C, maintaining said temperature for 6 hours (with sampling at certain time intervals to determine the kinetic reaction constant (k)). At the end of the resulting mixture was cooled and the crude product was discharged which, as inferred from NMR and GC analyses, was only composed of:

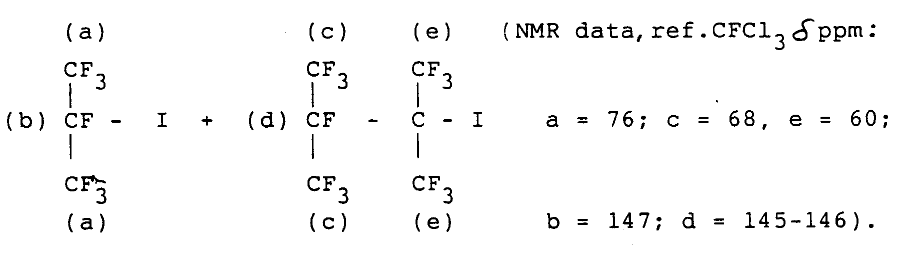

Analogous tests at different tempertures gave the same results and allowed to calculate the kinetic reaction constant as a function of the temperature:

| T°C | k |
|---|---|
| 151 | 2.3.10⁻⁵ |
| 161 | 7.4.10⁻⁵ |

### EXAMPLE 3

Following the procedure of the preceding example, 30 g (0.061 moles) of the branched perfluoroalkane used in example 2 and 16 g of I₂ (0.063 moles) were employed. At the end, the whole reaction mixture was cooled and the crude product was discharged. According to NMR analysis and GC analysis the mixture was constituted by the compounds in example 2.

### EXAMPLE 4

The autoclave used in example 1 was charged with 15 g of the branched perfluoroalkane used in example 2 (0.031 moles) and 14.8 g of Br₂ (0.092 moles). The resulting mixture was heated to 161°C and this temperature was maintained for 9 hours. At the end, a crude product was discharged which was composed of the following compounds:

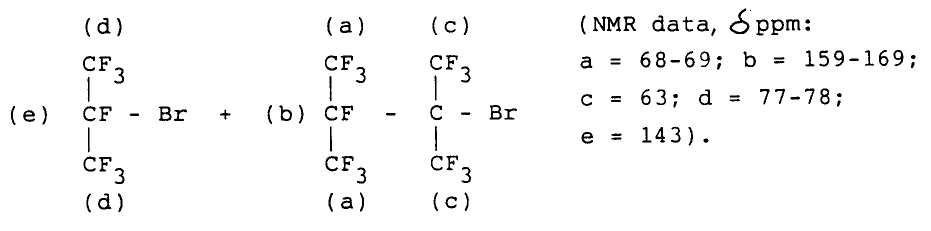

As in example 2, the kinetic constants at different temperatures were calculated:

| T°C | k |
|---|---|
| 141 | 7.0.10⁻⁶ |
| 151 | 2.3.10⁻⁵ |
| 161 | 7.6.10⁻⁵ |

### EXAMPLE 5

The reactor used in example 1 was charged with 15 g of perfluoroalkane (H) (0.031 moles) and 23.6 g of I₂ (0.093 moles). The resulting mixture was heated to 191°C and was allowed to stand for 8 hours. Thereafter a crude product was discharged which contained about 15% of the starting perfluoroalkane and the following compounds: 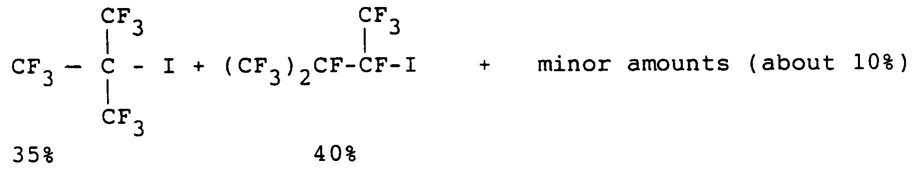
of:

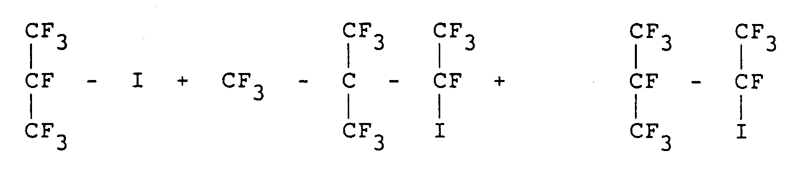

### EXAMPLE 6

The autoclave and the procedure of the preceding examples was used. 10 g of perfluoroalkane (G) (0.019 moles) and 14.2 g of I₂ (0.056 moles) were reacted at 150°C for 6 hours. At the end, the whole mixture was cooled and the crude reaction product was analyzed. Said product was composed, besides of about 10% of unreacted perfluoroalkane,of:

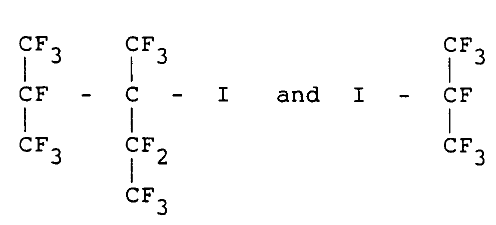

### EXAMPLE 7

A 50 ml autoclave made of AISI 316 steel was charged with 20 g of perfluoroalkane (F) (0.041 moles) and 8.6 g of Cl₂ (0.123 moles). The whole mixture was heated to 150° and maintained at this temperature for 10 hours, whereafter it was cooled and the crude reaction product was discharged. Said product contained the starting perfluoroalkane and the following compounds:

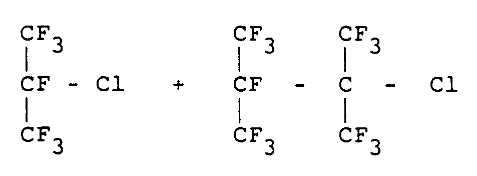

## Claims

1. Perfluoroalkyl halides wherein the carbon atom linked to the halogen atom is of the secondary or tertiary type, said halides having one of the general formulae (1) to (5): 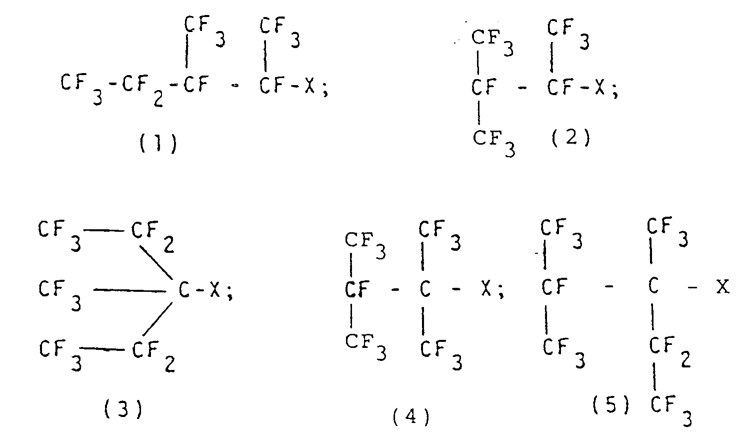 wherein X is I, Cl, Br; provided that in formula (2) X is only Br and that in formulae (3) and (4) X is not Br.

2. Perfluoroalkyl halides according to claim 1, i.e.,

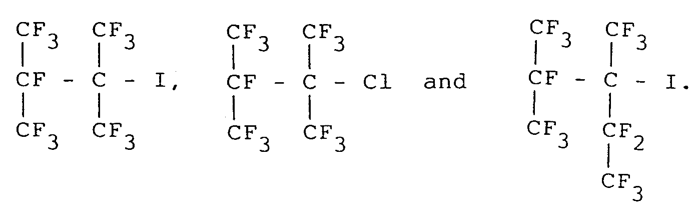

3. Process for preparing branched perfluoroalkyl halides wherein the carbon atom linked to the halogen atom is of the tertiary or secondary type, comprising the thermal decomposition, in the presence of a halogen of formula X₂ (X = Br, Cl or I) and at a temperature ranging from 90° to 250°C, of a branched perfluoroalkane containing at least one tertiary carbon atom and one quaternary carbon atom or two adjacent quaternary carbon atoms.

4. Process according to claim 3 for preparing the compound of formula: 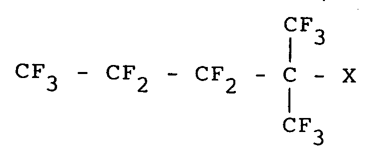 wherein X is as defined above, starting from the perfluoroalkane of formula:

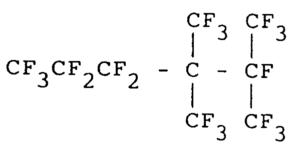

5. Process according to claim 3 for preparing the compound of formula: 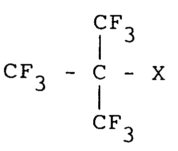 wherein X is as defined above, starting from one of the following perfluoroalkanes:

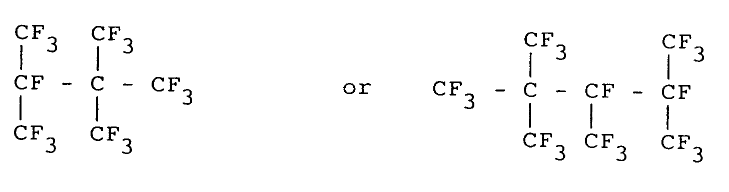

6. Process according to claim 3, wherein the compound of formula (1): 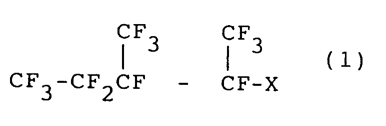 (X = I, Br or Cl)
is obtained by starting from the compound of formula: 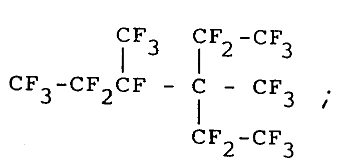 or the compound of formula (2): 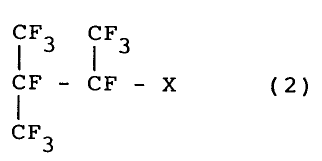 (X = I, Br or Cl)
is obtained by starting from the perfluoroalkane of formula: 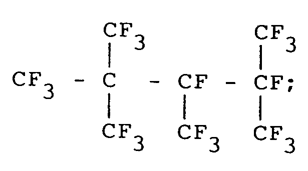 or the compound of formula (3): 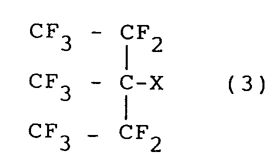 (X = I, Cl or Br)
is obtained by starting from the perfluoroalkane of formula: 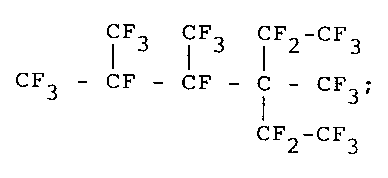 or the compound of formula (4): 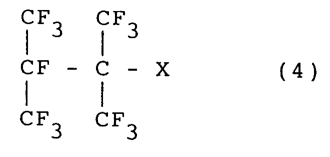 (X = I, Br or Cl)
is obtained by starting from the perfluoroalkane of formula: 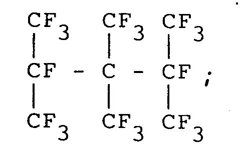 or the compound of formula (5): 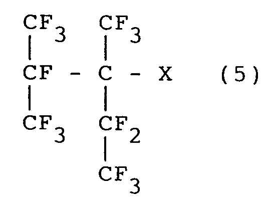 (X = I, Br or Cl)
is obtained by starting from the perfluoroalkane of formula:

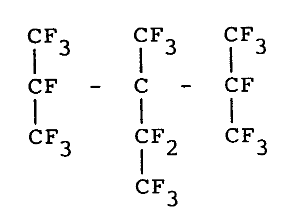

7. Process according to any one of claims 3 to 6 wherein the halogen is used in an at least stoichiometric amount with respect to the branched perfluoroalkane.

8. Process according to any one of claims 3 to 7, wherein the thermal decomposition is conducted at a temperature of from 130° to 180°C.

## Patentansprüche

1. Perfluoralkylhalogenide, in denen das mit dem Halogenatom verknüpfte Kohlenstoffatom vom sekundären oder tertiären Typ ist, wobei diese Halogenide eine der allgemeinen Formeln (1) bis (5) aufweisen: 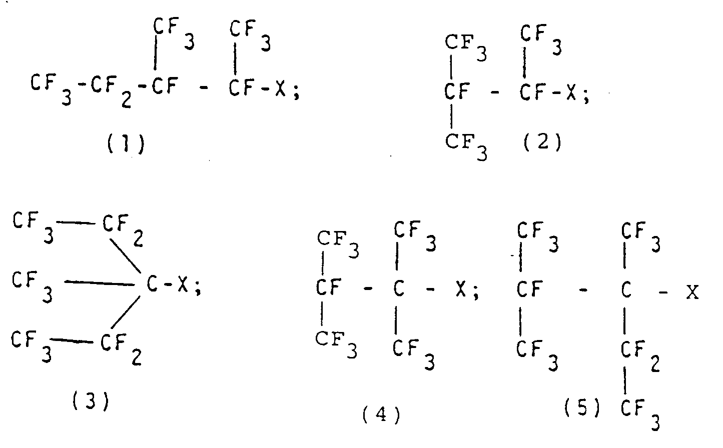 worin X für I, Cl, Br steht; mit der Maßgabe, daß in Formel (2) X nur Br ist und daß in den Formeln (3) und (4) X nicht für Br steht.

2. Perfluoralkylhalogenide nach Anspruch 1, d.h.

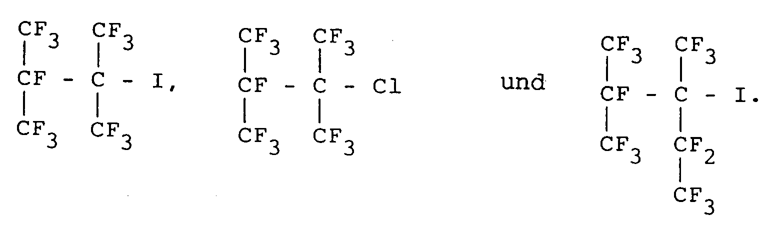

3. Verfahren zur Herstellung verzweigter Perfluoralkylhalogenide, in denen das mit den Halogenaton verknüpfte Kohlenstoffatom vom tertiären oder sekundären Typ ist, umfassend die thermische Zersetzung, in Anwesenheit eines Halogens X₂ (X = Br, Cl oder I) und bei einer Temperatur im Bereich von 90 bis 250°C, eines verzweigten Perfluoralkans, das wenigstens ein tertiäres Kohlenstoffatom und ein quaternäres Kohlenstoffatom oder zwei benachbarte quaternäre Kohlenstoffatome enthält.

4. Verfahren nach Anspruch 3 zur Herstellung der Verbindung der Formel: 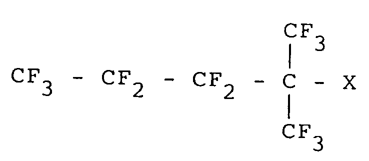 worin X wie oben definiert ist, ausgehend von dem Perfluoralkan der Formel:

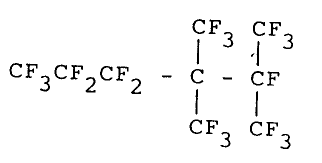

5. Verfahren nach Anspruch 3 zur Herstellung der Verbindung der Formel: 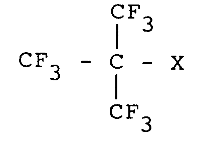 worin X wie oben definiert ist, ausgehend von einem der folgenden Perfluoralkane:

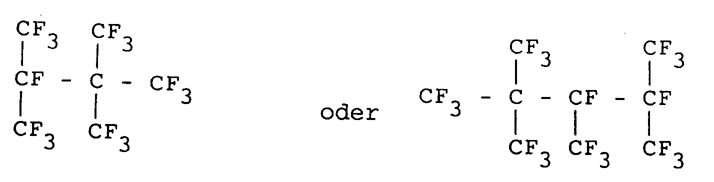

6. Verfahren nach Anspruch 3, in welchem die Verbindung der Formel (1): 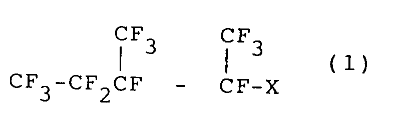 (X = I, Br oder Cl)
ausgehend von der Verbindung der Formel: 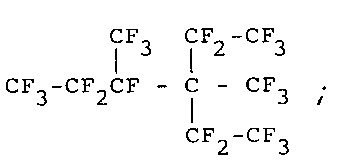 erhalten wird; oder die Verbindung der Formel (2): 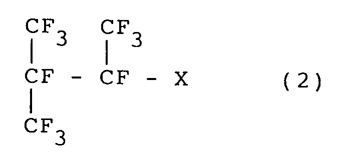 (X = I, Br oder Cl)
ausgehend von dem Perfluoralkan der Formel: 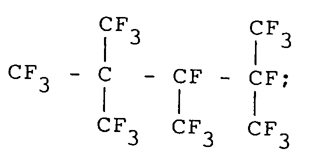 erhalten wird; oder die Verbindung der Formel (3): 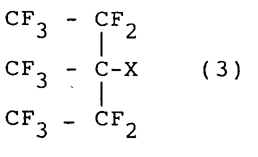 (X = I, Cl oder Br)
ausgehend von dem Perfluoralkan der Formel: 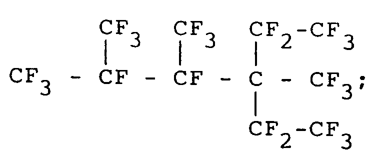 erhalten wird; oder die Verbindung der Formel (4): 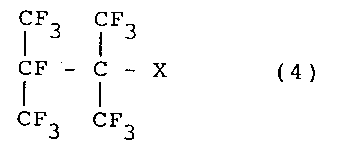 (X = I, Br oder Cl)
ausgehend von dem Perfluoralkan der Formel: 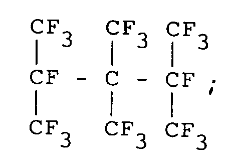 erhalten wird; oder die Verbindung der Formel (5): 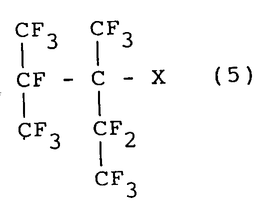 (X = I, Br oder Cl)
ausgehend von dem Perfluoralkan der Formel: 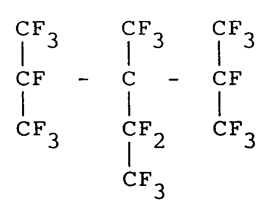 erhalten wird.

7. Verfahren nach irgendeinem der Ansprüche 3 bis 6, in welchem das Halogen bezogen auf das verzweigte Perfluoralkan in wenigstens stöchiometrischer Menge eingesetzt wird.

8. Verfahren nach irgendeinem der Ansprüche 3 bis 7, in welchem die thermische Zersetzung bei einer Temperatur von 130 bis 180°C durchgeführt wird.

## Revendications

1. Halogénures de perfluoroalkyle dans lesquels l'atome de carbone lié à l'atome d'halogène est du type secondaire ou du type tertiaire, ces halogénures répondant à l'une des formules générales (1) à (5) : 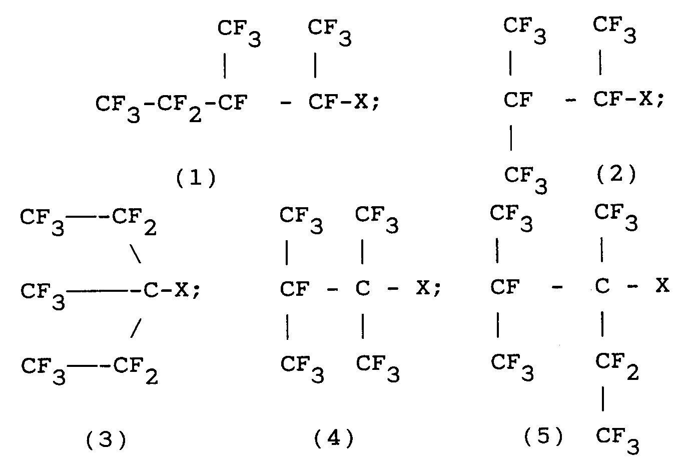 dans lesquelles
X représente I, Cl, Br; à condition que, dans la formule (2), X corresponde uniquement à Br et que, dans les formules (3) et (4), X ne corresponde pas Br.

2. Halogénures de perfluoroalkyle selon la revendication 1, c'est-à-dire:

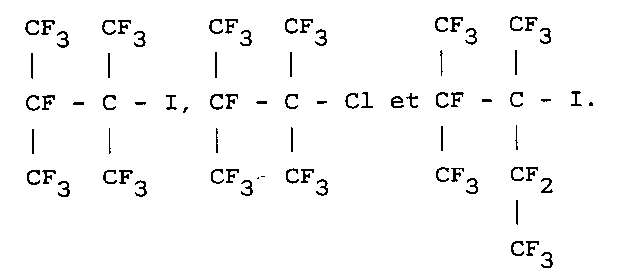

3. Procédé de préparation d'halogénures de perfluoroalkyle ramifié, dans lesquels l'atome de carbone lié à l'atome d'halogène est du type secondaire ou du type tertiaire, comprenant la décomposition thermique, en présence d'un halogène de formule X₂ (X = Br, Cl ou I), a une température comprise entre 90 et 250°C, d'un perfluoroalcane ramifié contenant au moins un atome de carbone tertiaire et un atome de carbone quaternaire ou deux atomes de carbone quaternaires adjacents.

4. Procédé selon la revendication 3 de préparation d'un dérivé de formule: 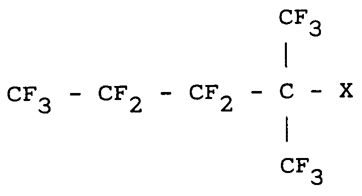 dans laquelle
X est tel que défini ci-dessus, à partir du perfluoroalcane de formule:

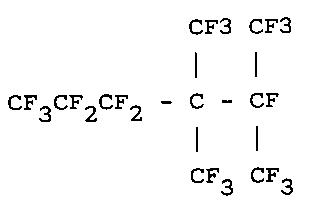

5. Procédé selon la revendication 3 de préparation d'un dérivé de formule: 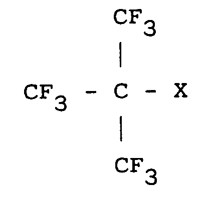 dans laquelle
X est tel que défini ci-dessus, à partir de l'un des perfluoroalcanes suivants:

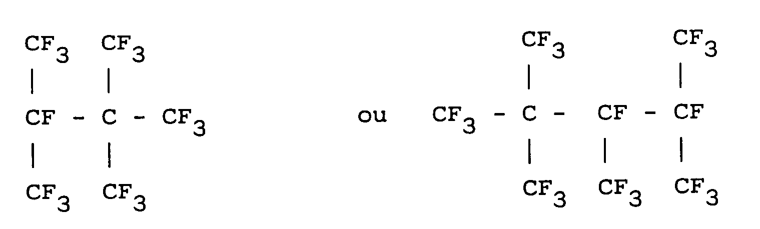

6. Procédé selon la revendication 3, caractérisé en ce que le produit de formule (1): 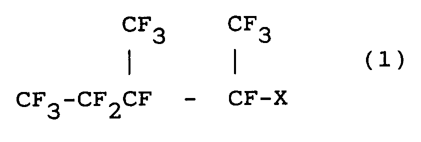 (X = I, Br ou Cl)
est obtenu à partir du dérivé de formule: 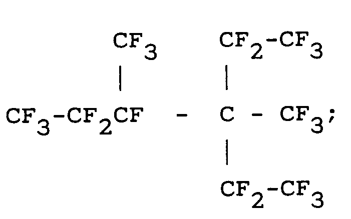 ou du dérivé de formule (2): 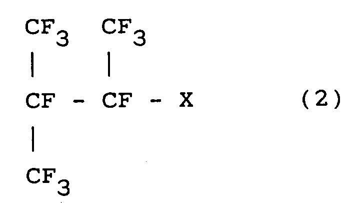 (X = I, Br ou Cl)
ou à partir du perfluoroalcane de formule: 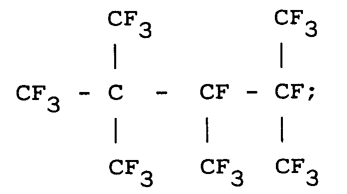 ou du dérivé de formule (3): 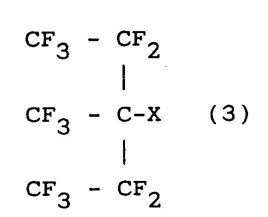 (X = I, Cl ou Br)
ou à partir du perfluoroalcane de formule: 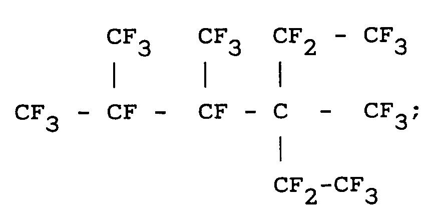 ou du dérivé de formule (4): 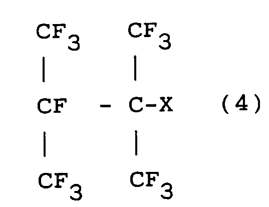 (X = I, Br ou Cl)
ou à partir du perfluoroalcane de formule: 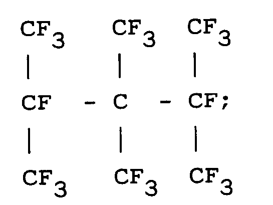 ou du dérivé de formule (5): 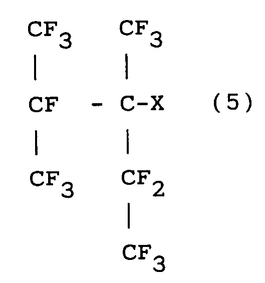 (X = I, Br ou Cl)
ou à partir du perfluoroalcane de formule:

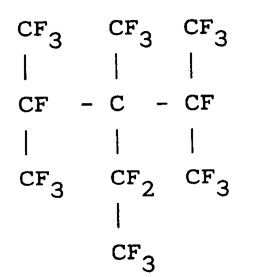

7. Procédé selon l'une quelconque des revendications 3 à 6 caractérisé en ce que l'on utilise l'halogène en quantité au moins stoechiométrique par rapport au perfluoroalcane ramifié.

8. Procédé selon l'une quelconque des revendications 3 à 7, caractérisé en ce que la décomposition thermique est réalisée à des températures de 130 à 180°C.
